# EUROPEAN PATENT SPECIFICATION

(11) **EP 1 812 367 B1**
(45) Date of publication and mention of the grant of the patent: **09.03.2011**
(21) Application number: 05824367.6
(22) Date of filing: 17.11.2005
(51) Int. Cl.: C07C 41/22, C07C 43/12

(54) **PROCESS FOR PRODUCTION OF 1,2,2,2-TETRAFLUORO ETHYL DIFLUORO METHYL ETHER**
VERFAHREN ZUR HERSTELLUNG VON 1,2,2,2-TETRAFLUOR-ETHYL-DIFLUOR-METHYLETHER
PROCEDE POUR LA FABRICATION DE L'ETHER DE 1,2,2,2-TETRAFLUOROETHYLE ET DE DIFLUOROMETHYLE

(30) Priority: 17.11.2004 US 628708 P
(43) Date of publication of application: 01.08.2007
(73) Proprietor: Minrad Inc., Orchard Park, NY 14127 (US)
(72) Inventor: TERRELL, Ross, C, New Port Richey, Florida 34653 (US); LEVINSON, Joshua, A., Zionsville, PA 18092 (US)
(74) Representative: HOFFMANN EITLE
(86) International application number: PCT/US2005/041754
(87) International publication number: WO 2006/055749

(56) References cited:
- GB-A- 2 219 292
- US-A- 5 026 924
- US-B1- 6 800 786
- PETROV ET AL: "Polyfluorinated carbocations stabilized by oxygen and sulfur" JOURNAL OF FLUORINE CHEMISTRY, ELSEVIER, NL, vol. 95, no. 1-2, 4 June 1999 (1999-06-04), pages 5-13, XP022576400 ISSN: 0022-1139

## Description

### FIELD OF THE INVENTION

The present invention is directed to the field of inhalation anesthetics. More specifically, this invention is directed to an improved process for the preparation of 1,2,2,2-tetrafluoroethyl difluoromethyl ether (Desflurane).

### BACKGROUND OF THE INVENTION

In general, the fluorination of alkanes and alkane-bearing compounds has been beset with the inability to efficiently monofluorinate a reaction substrate at a desired position such that reaction conversion (i.e., the proportion of substrate which does not remain unreacted) and yield (proportion of reacted substrate which undergoes fluorination) are high. In an attempt to obtain high yield and conversion, fluorinating agents have been used in molar amounts which are stoichiometrically in excess of reaction substrate. Even so, conversion and yield remain poor.

Excess solvent and fluorinating agent, as well as any off-products such as those arising from reaction substrate degradation (as opposed to fluorination), can remain as impurities in the desired fluorinated end-product and may require removal via purification.

The compound 1,2,2,2-tetrafluoroethyl difluoromethyl ether (CF₃CBFOCHF₂), a halogenated alkyl ether also known as Desflurane, is an important inhalation anesthetic, particularly suited for outpatient procedures or for conscious sedation. For this purpose, it must be highly pure. Economical and effective methods for the preparation of Desflurane which minimize or eliminate impurities are, therefore, highly desirable.

There are several known methods for the preparation of Desflurane. One method employs CF₃CHFOCH₃ as starting material, which is photochlorinated to give CF₃CHFOCHCl₂ This dichlorinated compound is then reacted with anhydrous HF in the presence of an antimony pentachloride (SbCl₅) catalyst to give CF₃CHFOCHF₂ (Desflurane). (See Ger. Offen 2,361,058 (1975)). This method suffers from the disadvantage in that it is a complex multistep synthesis using large amounts of chlorine, and, upon fluorination, produces, among other impurities, off-fluorinated products (such as CF₃CHFOCH₂F). The preparation of a sufficiently pure product is therefore cumbersome and expensive. The fact that some impurities are similar to the desired product except for their fluorination at off positions means that the desired product contains impurities of similar weight and structure to the desired product, leading to purification difficulties.

The use of hydrogen fluoride in the presence of antimony pentachloride has, nevertheless, been an attractive method for preparing many fluorinated hydrocarbons and their structurally-related derivatives. U.S. Patent No. 2,005,708, for example, describes the production of chlorofluoroalkanes from the reaction of a chlorinated hydrocarbon with hydrogen fluoride in the presence of antimony pentachloride or a mixture of antimony pentachloride and antimony trichloride. U.S. Patent No. 2,005,705 also describes fluorination of organochlorine compounds (e.g., carbon tetrachloride, methylene chloride, fluorotrichloromethane, etc.) with hydrogen fluoride in the presence of an antimony pentachloride catalyst to produce chlorofluoroalkanes.

It is also known that mixed halogenates can be successfully used as catalysts in the preparation of fluorinated alkane compounds. European Application No. 129,863 describes a process whereby antimony pentachloride is reacted with hydrogen fluoride to produce mixed halogenates, i.e., antimony chlorofluorides. The mixed halogenates are then reacted with a haloalkane (for example, carbon tetrachloride, as well as other chlorocarbons) to produce chlorofluoroalkanes.

The use of antimony pentachloride is particularly advantageous in the case of preparing fluorinated ethers in that it can make the use of high temperatures and pressures unnecessary. U.S. Patent No. 4,874,901, an example of a current ether fluorination method, describes the fluorination of CF₃CH₂OCHCl₂ to give CF₃CH₂OCHF₂ and CF₃CHClOCHCl₂ to give CF₃CHClOCHF₂ in the absence of antimony pentachloride. Elevated pressures as well as temperatures in excess of 200°C are required. U.S. Patent No. 3,869,519 describes the fluorination of CF₃(CH₃)CHOCHCl₂ with anhydrous hydrogen fluoride in the presence of antimony pentachloride to give CF₃(CH₃)CHOCHF₂. The potential use of antimony pentafluoride is disclosed as well. U.S. Patent No 3,962,460 describes the fluorination of several other chloroether compounds with anhydrous hydrogen fluoride in the presence of antimony pentachloride. The pressures and temperatures are significantly less than those required in U.S. Patent No. 4,874,901.

Of particular relevance to the formation of Desflurane, U.S. Patent No. 5,026,924 describes a process for fluorination of CF₃CHClOCHF₂ (isoflurane) to give CF₃CHFOCHF₂ (desflurane) using SbCl₅ as a catalyst. The patent also discloses an antimony chlorofluoride catalyst; indicating that "those skilled in the art are aware that hydrogen fluoride and antimony pentachloride react to form a mixed antimony fluorochloride catalyst in situ. Such a mixed catalyst would likewise be usable in the present invention and is contemplated here."

However, the process of U.S. Patent No. 5,026,924 suffers from a serious disadvantage in that it is necessary to use a substantial molar excess of hydrogen fluoride with respect to reaction substrate in order to achieve acceptable yields and conversions. The use of excess fluorinating agent adds to the cost of preparation, and can necessitate the removal of excess hydrogen fluoride from the Desflurane product. For example, this patent discloses the use of a 1.9:1 molar ratio of hydrogen fluoride to CF₃CHClOCHF₂ to obtain a conversion of 78.2% and a yield of 90.9%. However, with a 1:1 ratio of these reactants, the conversion was only 18% with a yield of 61%.

Journal of Fluorine Chemistry, Vol. 95, No. 1-2, pages 5-13 discloses the synthesis of desflurane by reacting SbF₅ and isoflurane without the co-presence of hydrogen fluoride. The desflurane product is synthesized at a conversion rate of 62.5%.

### SUMMARY OF THE INTENTION

The present invention is a method for the preparation of 1,2,2,2-tetrafluoroethyl difluoromethyl ether (CF₃CHFOCHF₂, Desflurane) which produces a high yield of product without requiring a molar excess of hydrogen fluoride fluorinating agent with respect to the reaction substrate.

Accordingly, the present invention provides a process for preparing CF₃CHFOCF₂H by reacting a mixture comprising CF₃CHClOCHF₂, antimony pentafluoride and 0.7-1.5 moles of hydrogen fluoride per mole of CF₃CHClOCHF₂.

Surprisingly, it has been found that in the preparation of (CF₃CHFOCHF₂), the use of antimony pentafluoride instead of antimony pentachloride or mixed antimony chlorofluoride catalysts can achieve good conversions and yields while avoiding the necessity of using a molar excess of hydrogen fluoride or the use of high reaction temperatures and pressures. Thus, the need to remove degradation products and recover and/or neutralize unreacted fluorinating agent is also avoided, resulting in a substantial economic advantage over currently practiced methods of preparation.

### DESCRIPTION OF THE INVENTION

In the process of the present invention, the starting material, 1-chloro-2,2,2-trifluoroethyl difluoromethyl ether (CF₃CHClOCHF₂) is reacted with hydrogen fluoride in the presence of antimony pentafluoride to produce 1,2,2,2-tetrafluoroethyl difluoromethyl ether (CF₃CHFOCHF₂).

The starting material for the process, 1-chloro-2,2,2,-trifluoroethyl difluoromethyl ether (CF₃CHClOCHF₂ or isoflurane) is commercially available. It can also be synthesized by the method described in U.S. Patent No. 3,535,388.

The hydrogen fluoride used in the reaction is preferably of commercial grade. Impurities (including water) may be present as long as they do not appreciably impair the conversions and yields of the fluorination reaction to produce 1,2,2,2-tetrafluoroethyl difluoromethyl ether. However, it is preferred that a high purity product be used, having an impurity content of less than about 1 wt% and which is essentially anhydrous (i.e., water is present in only trace amounts, if at all). Water can react with and destroy antimony halide catalysts. While excess catalyst can be added to the reaction to compensate for the loss of catalyst through reaction with water, catalyst degradation produces impurities which may need to be removed from the Desflurane final product. Hydrogen fluoride can be used as either as a gas, a liquid, or both.

Antimony pentafluoride is available commercially from specialty chemical manufacturers. Impurities, including water, may be present, but they may impair the achievement of appreciable yield of 1,2,2,2-tetrafluoroethyl difluoromethyl ether. It is preferred that the antimony pentafluoride catalyst be substantially pure at levels of about 99% purity or greater and be essentially anhydrous. With higher impurity content, an appreciable decline in conversion and yields may occur.

The reaction can be performed at a relatively low temperature, such as at one or more temperatures in the range of from -10 °C to 30 °C. A preferred reaction temperature range is from -7°C to 18 °C. Temperatures outside of the above ranges may be used. However, a decrease in reaction rate and in yield may result. Heating and cooling may be provided using a variety of techniques known in the art (e.g., a jacketed vessel with a temperature-controlled heat transfer fluid).

The reaction is conveniently performed at atmospheric pressure. If desired, a condenser can be used to trap and return reactant and product material, and a scrubber can be used to capture the acid by-products evolved during the reaction. However, the fluorination reaction can be performed at pressures higher than atmospheric, such as with a closed vessel operation mode, and the high yield, high conversion benefits of the present invention can still be obtained.

The molar ratio of hydrogen fluoride to CF₃CHClOCHF₂ is in the range of 0.7:1 to 1.5:1 and is more preferably in the range of 0.7:1 to 1.1:1. These ratios avoid the use of a large stoichiometric excess of hydrogen fluoride and the associated costs of recovery and/or neutralization. However, the use of ratios outside the above ranges will also give the high conversion, high yield benefits of the present invention. Note that these ratios apply whether the reactants are combined at once, as in a batch process, or over time.

The quantity of antimony pentafluoride is preferably in the range of 0.1 to 10 weight percent (relative to the weight of CF₃CHClOCHF₂) and is more preferably around 2 to 2.5 weight percent. However, the use of ratios outside the above ranges can also be used.

A solvent may be added to the reaction mixture, although it is not preferable to do so. A wide range of solvents can be used. If a solvent is used, it is preferred that the solvent does not react with other reaction components or otherwise appreciably impair the high yield of 1,2,2,2-tetrafluoroethyl difluoromethyl ether.

The reaction and subsequent purification can be carried out using a number of methods known in the art. One preferable method is to mix the antimony pentafluoride catalyst with the CF₃CHClOCHF₂ and then to add anhydrous hydrogen fluoride with stirring at a rate at which it reacts (i.e., a semi-continuous batch operation). The HCl liberated by the reaction can be removed by passing it through a chilled heat exchanger to a water scrubber. The course of the reaction can be followed by titration of the evolved HCl gas or by the addition, over time, of the HF. When the reaction is complete, the CF₃CHFOCHF₂ product can be isolated by washing the reaction mixture with water or dilute aqueous NaOH and can be subsequently purified by fractional distillation. Other modes of reaction include a batch operation (where the entire quantity of reactants and catalyst are added to the reaction vessel at the beginning) and a continuous operation (where the reactants and catalyst are fed continuously into the reaction vessel while product is withdrawn at an appropriate rate). Other purification modes may include vapor phase chromatography, extraction, absorption, and stripping.

The fluorination of isoflurane to produce desflurane has such high conversion and yield in the presence of antimony pentafluoride that even when the reactants (isoflurane and fluorinating agent) are only combined at the rate at which they react, the conversion and yield benefits are not lost. Thus, even when the hydrogen fluoride is present in very small amounts (much less than stoichiometric, such as when the fluorinating agent is added to the isoflurane at a rate at which the added fluorinating agent is used up immediately), high conversion and yield are still observed if the hydrogen fluoride is added until the reaction is complete. For example, at 15 - 20 °C and using 1 wt % SbF₅ as a catalyst (based upon the weight of isoflurane charged to the reaction vessel), the rate of Desflurane formation is approximately 0.2 mole per hour per mole isoflurane charged to the reaction vessel. When HF is added at this rate or lower, unreacted HF in the reaction vessel is avoided, and the wasting of hydrogen fluoride via spillover into the scrubber is avoided. Reaction times are generally in the range of from 1 to 15 hours. At reaction temperatures in the range of from -10 to 30°C, reaction rates (approximately equal to Desflurane formation rates) are generally in the range of from approximately 0.05 to 0.3 mole per hour per mole isoflurane charged to the reaction vessel, and more preferably in the range of from 0.15 to 0.25 mole per hour per mole isoflurane charged to the reaction vessel.

By use of the above method, CF₃CHFOCHF₂ can be prepared in yields of at least 50% and often in the range of from 60 or 70% to 99 % or even greater, with conversions of at least 10 %, but as high as 60 to 90%. The following examples more fully illustrate the practice of embodiments of the present invention. Such examples are for illustrative purposes and are not intended to limit the scope of the invention. Note that the calculated conversions and yields are defined as specified in U.S. Patent No. 5,026,924; "conversion" refers to the number of moles of CF₃CHClOCHF₂ reacted divided by the number of moles of CF₃CHClOCHF₂ charged to the reactor, and yield is the number of moles of CF₃CHFOCHF₂ formed divided by the number of moles of CF₃CHClOCHF₂ reacted. The "number of moles of CF₃CHClOCHF₂ reacted" includes the moles of CF₃CHClOCHF₂ which form products other than CF₃CHFOCHF₂. The components of the recovered material are expressed in weight percentage based on the total weight of the recovered material. As can be seen by the weight percentages, the components of recovered material are almost exclusively CF₃CHFOCHF₂ and CF₃CHClOCHF₂. It should be noted that in the examples below, due to "open air" manipulations of the Desflurane during purification, etc., some Desflurane is lost through evaporation due to its low boiling point.

### EXAMPLE 1 (comparative example)

184 g of CF₃CHClOCHF₂ (1 mole) was mixed with 4.4 g of SbF₅ (2.4% by weight), and anhydrous hydrogen fluoride was added while stirring the reaction mixture at 15-25° C. The effluent HCl gas formed in the reaction was led to a water scrubber via a "Dry Ice" condenser in order to monitor the course of the reaction and in order to determine when the reaction was complete. There was a slow but steady endothermic reaction. 12 g of hydrogen fluoride (0.6 moles) was added over a period of about six hours. At the end of this time, no additional HCl was collected in the scrubber, indicating that all of the added HF had reacted. The recovered material was washed with water to give 160 g, which was analyzed by gas chromatography. There was about 62% CF₃CHFOCHF₂ and about 38% CF₃CHClOCHF₂ (i.e., unreacted starting material) with four minor components all at 0.05% or less. The moles of CF₃CHCFOCHF₂ calculated from the gas chromatograph were 0.59 moles. This represents a conversion of 67% and a yield of 88% based on the starting material reacted. The ratio of HF to CF₃CHClOCHF₂ in this example was 0.6:1, giving a conversion of 67% and a yield of 88%, which is definitely superior to the conversion and yield reported in US. Patent No. 5,026,924, where a 0.5:1 ratio of HF to CF₃CHClOCHF₂ gave a conversion of 15% and a yield of 48%.

### EXAMPLE 2

239 g of CF₃CHClOCHF₂ (1.30 moles) was mixed with 6 g of SbF₅ (2.5% by weight), and anhydrous hydrogen fluoride was added at 15-20 °C. The effluent from the reaction was led to a water scrubber via a "Dry Ice" condenser in order to collect and titrate the HCl formed in the reaction. The reaction was slow, steady, and endothermic. After 1.03 moles of HCl had been evolved from the reaction (as determined by the titration of the water scrubber using standard NaOH), the reaction was terminated. The reaction mixture was washed with ice water to give 203 g. This was analyzed by gas chromatography and was shown to be 76% CF₃CHFOCHF₂ and 23% unreacted starting material (CF₃CHClOCHF₂). The conversion calculated from the gas chromatogram was 80.5% and the yield 87.8% based on the starting material reacted. Therefore, the ratio of HF to CF₃CHClOCHF₂ in this example was 0.79:1, giving a conversion of 80.5% and yield of 87.8%. By comparison, U.S. Patent No. 5,026,924 used a 1:1 ratio of HF to CF₃CHClOCBF₂ that gave a conversion of 18% and a yield of 61 %.

### EXAMPLE 3 (comparative example)

156 g of CF₃CHClOCHF₂ (0.85 moles) was mixed with 3.9 g of SbF₅ (2.5% by weight), and anhydrous hydrogen fluoride was added at 0-30 °C. The effluent from the reaction was led to a water scrubber via a "Dry ice" condenser in order to collect and titrate the HCl formed in the reaction. The reaction was slow, steady, and endothermic. After 0.5 moles of HCl were titrated in the scrubber, the reaction was terminated. The product weighed 140 g. There was very little residual acid in the product as determined by titration with standard base. The product was analyzed by gas chromatography and was shown to be 52% CF₃CHFOCHF₂ and 48% unreacted starting material (CF₃CHClOCHF₂). The conversion calculated from the chromatogram was 57%, and the yield was 89.3%. The ratio of HF to CF₃CHClOCHF₂ in this example was 0.59:1, giving a conversion of 57% and a yield of 89.3%. These results are superior to those reported in U.S. Patent No. 5,026,924, where a 0.5:1 ratio of HF to CF₃CHClOCHF₂ gave a conversion of 15% and a yield of 48%.

### EXAMPLE 4

116 g of CF₃CHClOCHF₂ (0.63 mole) was mixed with 2.9 g of SbF₅ (2.5% by weight), and anhydrous hydrogen fluoride was added at 15-20 °C. The effluent from the reaction was led to a water scrubber via a "Dry Ice" condenser in order to collect and titrate the HCl formed in the reaction. The reaction was slow, steady, and endothermic. After 0.5 moles of hydrogen fluoride had been added and after the reaction was completed (as shown by no additional evolution of HCl into the scrubber), the reaction was terminated. The reaction product weighed 107 g and was 74% CF₃CHFOCHF₂ and 25% unreacted CF₃CHClOCHF₂. The conversion was 77%, and the yield was almost quantitative at 97.6% based on the amount of starting material reacted. The ratio of HF to CF₃CH₂ClOCHF₂ used was 0.79:1. In this example, a 0.79:1 ratio of HF to CF₃CHClOCHF₂ gave a conversion of 77% and a nearly quantitative yield of 97.6%. U.S. Patent No. 5,026,924 used a 1:1 ratio of reactants that gave a smaller conversion of 18% and a smaller yield of 61 %.

### EXAMPLE 5

184 g of CF₃CHClOCHF₂ (1 mole) was mixed with 4.6 g of SbF₅ (2.5% by weight), and anhydrous hydrogen fluoride was added at 15-20 °C. The effluent from the reaction was led to a water scrubber via a Dry Ice" condenser in order to collect and titrate the HCl formed in the reaction. The reaction was slow, steady, and endothermic. After 0.79 moles of acid had been collected in the scrubber, the reaction was terminated. The crude reaction product was washed with water to give 157 g, which was 84% CF₃CHFOCHF₂ and 15% CF₃CHClOCHF₂ as determined by gas chromatography. The conversion was 87.2%, the yield was 90%, and the ratio of HF to CF₃CHClOCHF₂ used in this example was 0.79:1. U.S. Patent 5,026,924 gave a conversion of 18% and a yield of 61% using a higher HF to CF₃CHClOCHF₂ ratio at 1:1.

### EXAMPLE 6

147 g of CF₃CHClOCHF₂ (0.8 moles) was mixed with 1.5 g of SbF₅ (1.0% by weight, which is a lower proportion than the quantities used in the previous five examples), and anhydrous hydrogen fluoride was added at 15-20 °C. The effluent from the reaction was led to a water scrubber via a "Dry ice" condenser in order to collect and titrate the HCl formed in the reaction. The reaction was slow, steady, and endothermic. After 0.64 moles of acid had been collected in the scrubber, the reaction was terminated. The crude reaction product was washed with water and was found to contain 0.15 moles of acid. The organic product collected was 135 g and contained 85.5% a CF₃CHFOCHF₂ and 13.7% CF₃CHClOCHF₂ as determined by gas chromatography with six minor components all at 0.2% or less. The conversion was 87.5%, the yield was 98.1 %, and the ratio of HF to CF₃CHClOCHF₂ used in this example was approximately 0.99:1. U.S. Patent 5,026,924 gave a lower conversion of 18% and a lower yield of 61 % using a slightly higher HF to CF₃CHClOCHF₂ ratio at 1:1.

### EXAMPLE 7

184 g of CF₃CHClOCHF₂ (1.0 mole) was mixed with 3.9 g of SbF₅ (2.1% by weight), and anhydrous hydrogen fluoride was added at 15-20 °C. The effluent from the reaction was led to a water scrubber via a "Dry ice" condenser in order to collect and titrate the HCl formed in the reaction. The reaction was slow, steady, and endothermic. After 0.65 moles of acid had been collected in the scrubber, the reaction was terminated. The crude reaction product was washed with water and was found to contain 0.05 moles of acid. The organic product collected was 147 g and contained 60% CF₃CHFOCHF₂ and 40% CF₃CHClOCHF₂ as determined by gas chromatography. The conversion was 68%, the yield was 77%, and the ratio of HF to CF₃CHClOCHF₂ used in this example was approximately 0.7:1. These results are superior to those reported in U.S. Patent No. 5,026,924, where a 0.5:1 ratio of HF to CF₃CHClOCHF₂ gave a conversion of 15% and a yield of 48%.

### EXAMPLE 8

184 g of CF₃CHClOCHF₂ (1.0 mole) was mixed with 4 g of SbF₅ (2.2% by weight), and anhydrous hydrogen fluoride was added at 15-20 °C. The effluent from the reaction was led to a water scrubber via a "Dry ice" condenser in order to collect and titrate the HCl formed in the reaction. The reaction was slow, steady, and endothermic. After 0.89 moles of acid had been collected in the scrubber, the reaction was terminated. The crude reaction product was washed with water and was found to contain 0.08 moles of acid. The organic product collected was 142 g and contained 82% CF₃CHFOCHF₂ and 17% CF₃CHClOCHF₂ as determined by gas chromatography. The conversion was 87%, the yield was 80%, and the ratio of HF to CF₃CHClOCHF₂ used in this example was approximately 0.97:1. U.S. Patent 5,026,924 gave a lower conversion of 18% and a lower yield of 61% using a 1:1 ratio of HF to CF₃CHClOCHF₂.

### EXAMPLE 9

184 g of CF₃CHClOCHF₂ (1.0 mole) was mixed with 1.84 g of SbF₅ (1% by weight), and anhydrous hydrogen fluoride was added at 15-20 °C. The effluent from the reaction was led to a water scrubber via a "Dry ice" condenser in order to collect and titrate the HCl formed in the reaction. The reaction was slow, steady, and endothermic. After 0.8 moles of acid had been collected in the scrubber, the reaction was terminated. The crude reaction product was washed with water and was found to contain 0.05 moles of acid. The organic product collected was 145 g and contained 60% CF₃CHFOCHF₂ and 40% CF₃CHClOCBF₂ as determined by gas chromatography. The conversion was 68.5%, the yield was 76%, and the ratio of HF to CF₃CHClOCHF₂ used in this example was approximately 0.85:1. U.S. Patent 5,026,924 gave a lower conversion of 18% and a lower yield of 61% using a 1:1 ratio ofHF to CF₃CHClOCHF₂.

### EXAMPLE 10

184 g of CF₃CHClOCHF₂ (1.0 mole) was mixed with 2.76 g of SbF₅ (1.5% by weight), and anhydrous hydrogen fluoride was added at 15-20 °C. The effluent from the reaction was led to a water scrubber via a "Dry ice" condenser in order to collect and titrate the HCl formed in the reaction. The reaction was slow, steady, and endothermic. After 0.91 moles of acid had been collected in the scrubber, the reaction was terminated. The crude reaction product was washed with water and was found to contain 0.05 moles of acid. The organic product collected was 143 g and contained 75% CF₃CHFOCHF₂ and 25% CF₃CHClOCHF₂ as determined by gas chromatography. The conversion was 80.6%, the yield was 79%, and the ratio of HF to CF₃CHClOCHF₂ used in this example was approximately 0.96:1. U.S. Patent 5,026,924 gave a lower conversion of 18% and a lower yield of 61% using a 1:1 ratio of HF to CF₃CHClOCHF₂.

### EXAMPLE 11

184 g of CF₃CHClOCBF₂ (1.0 mole) was mixed with 2.76 g of SbF₅ (1.5% by weight), and anhydrous hydrogen fluoride was added at 15-20 °C. The effluent from the reaction was led to a water scrubber via a "Dry ice" condenser in order to collect and titrate the HCl formed in the reaction. The reaction was slow, steady, and endothermic. After 0.9 moles of acid had been collected in the scrubber, the reaction was terminated. The crude reaction product was washed with water and was found to contain 0.06 moles of acid. The organic product collected was 148 g and contained 73% CF₃CHFOCHF₂ and 27% CF₃CHClOCHF₂ as determined by gas chromatography. The conversion was 78%, the yield was 82%, and the ratio of HF to CF₃CHClOCHF₂ used in this example was approximately 0.96:1. U.S. Patent 5,026,924 gave a lower conversion of 18% and a lower yield of 61 % using a 1:1 ratio of HF to CF₃CHClOCHF₂.

### EXAMPLE 12

184 g of CF₃CHClOCBF₂ (1.0 mole) was mixed with 2.76 g of SbF₅ (1.5% by weight), and anhydrous hydrogen fluoride was added at 15-20 °C. The effluent from the reaction was led to a water scrubber via a "Dry ice" condenser in order to collect and titrate the HCl formed in the reaction. The reaction was slow, steady, and endothermic. After 0.94 moles of acid had been collected in the scrubber, the reaction was terminated. The crude reaction product was washed with water and was found to contain 0.05 moles of acid. The organic product collected was 140 g and contained 62% CF₃CHFOCHF₂ and 37% CF₃CHClOCHF₂ as determined by gas chromatography. The conversion was 72%, the yield was 72%, and the ratio of HF to CF₃CHClOCHF₂ used in this example was approximately 0.99:1. U.S. Patent 5,026,924 gave a lower conversion of 18% and a lower yield of 61% using a 1:1 ratio of HF to CF₃CHClOCHF₂.

## Claims

1. A process for preparing CF₃CHFOCF₂H by reacting a mixture comprising CF₃CHClOCHF₂, antimony pentafluoride and 0.7-1.5 moles of hydrogen fluoride per mole of CF₃CHClOCHF₂.

2. A process according to Claim 1, wherein 0.7-1.1 moles of hydrogen fluoride are added per mole of CF₃CHClOCHF₂ charged to the reaction vessel.

3. A process according to Claim 1, wherein the temperature of the reaction is in the range -10°C to 30°C.

4. A process according to Claim 1, wherein the temperature of the reaction is in the range -7°C to 18°C.

5. A process according to Claim 1, wherein the mixture is formed by:
a) combining CF₃CHClOCHF₂ with antimony pentafluoride; and
b) adding hydrogen fluoride to said combined CF₃CHClOCHF₂ and antimony pentafluoride while stirring.

6. A process according to Claim 1, wherein the yield of CF₃CHFOCF₂H is 60% or greater.

7. A process according to Claim 6, wherein the reaction conversion is 60% or greater.

8. A process according to Claim 1, wherein the quantity of antimony pentafluoride is in the range of from 0.1 to 10 weight percent relative to the weight of CF₃CHClOCHF₂.

9. A process according to Claim 8, wherein the molar amount of hydrogen fluoride added per mole of CF₃CHClOCHF₂ is in the range 0.7-1.1:1 and the quantity of antimony pentafluroide is in the range of from 2 to 2.5 weight % relative to the weight of CF₃CHClOCHF₂.

10. A process according to Claim 8, wherein CF₃CHFOCF₂H is separated out from the reaction mixture.

11. A process according to Claim 1 comprising:
a) forming a mixture comprising CF₃CHClOCHF₂, antimony pentafluoride and hydrogen fluoride; and
b) adding hydrogen fluoride to the mixture at a rate approximately equal to or less than the rate at which the hydrogen fluoride reacts with the CF₃CHClOCHF₂ to form CF₃CHFOCF₂H.

12. A process according to Claim 11, wherein the hydrogen fluoride is added at a rate of 0.10 to 0.30 moles per hour per mole of CF₃CHClOCHF₂ charged to the reaction vessel.

13. A process according to Claim 12, wherein the hydrogen fluoride is added at a rate of 0.15 to 0.25 moles per hour per mole of CF₃CHClOCHF₂ charged to the reaction vessel.

14. A process according to Claim 11, which provides both a yield and conversion of above 70%.

## Patentansprüche

1. Verfahren zur Herstellung von CF₃CHFOCF₂H durch Umsetzen einer Mischung, welche CF₃CHClOCHF₂, Antimonpentafluorid und 0,7 bis 1,5 mol Wasserstofffluorid pro Mol CF₃CHClOCHF₂ umfasst.

2. Verfahren gemäss Anspruch 1, wobei 0,7 bis 1,1 mol Wasserstofffluorid pro Mol CF₃CHClOCHF₂, mit welchem das Reaktionsgefäss beladen ist, zugegeben wird.

3. Verfahren gemäss Anspruch 1, wobei die Reaktionstemperatur im Bereich von -10 bis 30°C liegt.

4. Verfahren gemäss Anspruch 1, wobei die Reaktionstemperatur im Bereich von -7 bis 18°C liegt.

5. Verfahren gemäss Anspruch 1, wobei die Mischung gebildet wird durch:
(a) Vereinen von CF₃CHClOCHF₂ mit Antimonpentafluorid; und
(b) Zugabe von Wasserstofffluorid unter Rühren zu dem gemischten CF₃CHClOCHF₂ und Antimonpentafluorid.

6. Verfahren gemäss Anspruch 1, wobei die Ausbeute an CF₃CHFOCF₂H 60 % oder mehr ist.

7. Verfahren gemäss Anspruch 6, wobei der Reaktionsumsatz 60 % oder mehr ist.

8. Verfahren gemäss Anspruch 1, wobei die Menge an Antimonpentafluorid im Bereich von 0,1 bis 10 Gew.%, bezogen auf das Gewicht von CF₃CHClOCHF₂, liegt.

9. Verfahren gemäss Anspruch 8, wobei die molare Menge an Wasserstofffluorid, welche pro Mol CF₃CHClOCHF₂ zugegeben wird, im Bereich von 0,7 bis 1,1:1 liegt und die Menge an Antimonpentafluorid im Bereich von 2 bis 2,5 Gew.%, bezogen auf das Gewicht von CF₃CHClOCHF₂, liegt.

10. Verfahren gemäss Anspruch 8, wobei CF₃CHFOCF₂H von der Reaktionsmischung abgetrennt wird.

11. Verfahren gemäss Anspruch 1, umfassend:
(a) Bilden einer Mischung, welche CF₃CHClOCHF₂, Antimonpentafluorid und Wasserstofffluorid umfasst; und
(b) Zugabe von Wasserstofffluorid zu der Mischung in einer Geschwindigkeit, die ungefähr gleich oder kleiner als die Geschwindigkeit ist, bei der Wasserstofffluorid mit CF₃CHClOCHF₂ reagiert und CF₃CHFOCF₂H bildet.

12. Verfahren gemäss Anspruch 11, wobei Wasserstofffluorid in einer Geschwindigkeit von 0,10 bis 0,30 mol/std pro Mol CF₃CHClOCHF₂, mit welchem das Reaktionsgefäss beladen ist, zugegeben wird.

13. Verfahren gemäss Anspruch 12, wobei Wasserstofffluorid in einer Geschwindigkeit von 0,15 bis 0,25 mol/std pro Mol CF₃CHClOCHF₂, mit welchem das Reaktionsgefäss beladen ist, zugegeben wird.

14. Verfahren gemäss Anspruch 11, welches eine Ausbeute und einen Umsatz von über 70 % liefert.

## Revendications

1. Processus de préparation de CF₃CHFOCF₂H en faisant réagir un mélange comprenant du CF₃CHClOCHF₂, du pentafluorure d'antimoine et 0,7 à 1,5 moles de fluorure d'hydrogène par mole de CF₃CHClOCHF₂.

2. Processus selon la revendication 1, dans lequel 0,7 à 1,1 moles de fluorure d'hydrogène sont ajoutés par mole de CF₃CHClOCHF₂ chargé dans la cuve de réaction.

3. Processus selon la revendication 1, dans lequel la température de la réaction se trouve dans la plage de -10°C à 30°C.

4. Processus selon la revendication 1, dans lequel la température de la réaction se trouve dans la plage de -7°C à 18°C.

5. Processus selon la revendication 1, dans lequel le mélange est forme :
a) en combinant le CF₃CHClOCHF₂ avec le pentafluorure d'antimoine ; et
b) en ajoutant du fluorure d'hydrogène auxdits CF₃CHClOCHF₂ et pentafluorure d'antimoine combinés tout en agitant.

6. Processus selon la revendication 1, dans lequel le rendement en CF₃CHFOCF₂H est de 60% ou plus.

7. Processus selon la revendication 6, dans lequel la conversion de réaction est de 60% ou plus.

8. Processus selon la revendication 1, dans lequel la quantité de pentafluorure d'antimoine se trouve dans la plage de 0,1 à 10% en poids par rapport au poids du CF₃CHClOCHF₂.

9. Processus selon la revendication 8, dans lequel la quantité molaire de fluorure d'hydrogène ajoutée par mole de CF₃CHClOCHF₂ se trouve dans la plage de 0,7 à 1,1:1 1 et la quantité de pentafluorure d'antimoine se trouve dans la plage de 2 à 2,5% en poids par rapport au poids du CF₃CHClOCHF₂.

10. Processus selon la revendication 8, dans lequel le CF₃CHFOCF₂H est séparé du mélange réactionnel.

11. Processus selon la revendication 1, comprenant le fait :
a) de former un mélange comprenant du CF₃CHClOCHF₂, du pentafluorure d'antimoine et du fluorure d'hydrogène ; et
b) d'ajouter du fluorure d'hydrogène au mélange à une vitesse approximativement inférieure ou égale à la vitesse à laquelle le fluorure d'hydrogène réagit avec le CF₃CHClOCHF₂ pour former le CF₃CHFOCF₂H.

12. Processus selon la revendication 11, dans lequel le fluorure d'hydrogène est ajouté à une vitesse de 0,10 à 0,30 moles par heure par mole de CF₃CHClOCHF₂ chargé dans la cuve de réaction.

13. Processus selon la revendication 12, dans lequel le fluorure d'hydrogène est ajouté à une vitesse de 0,15 à 0,25 moles par heure par mole de CF₃CHClOCHF₂ chargé dans la cuve de réaction.

14. Processus selon la revendication 11, lequel fournit à la fois un rendement et une conversion au-dessus de 70%.
